(19) Europäisches Patentamt
European Patent Office
Office européen des brevets

(11) **EP 1 977 755 B1**

(12) **EUROPEAN PATENT SPECIFICATION**

(45) Date of publication and mention of the grant of the patent:
**09.10.2013 Bulletin 2013/41**

(51) Int Cl.:
*A61K 39/35* (2006.01)    *A61P 37/00* (2006.01)
*C07K 14/435* (2006.01)    *C07K 16/18* (2006.01)
*G01N 33/68* (2006.01)

(21) Application number: **08250659.3**

(22) Date of filing: **27.02.2008**

(54) **Shrimp allergen antishrimp allergen antibody and use thereof**

Garnelenallergen, Antikörper gegen Garnelenallergen und seine Verwendung

Allergène anti-crevette, anticorps allergène anti-crevette et leurs utilisations

(84) Designated Contracting States:
AT BE BG CH CY CZ DE DK EE ES FI FR GB GR HR HU IE IS IT LI LT LU LV MC MT NL NO PL PT RO SE SI SK TR

(30) Priority: **05.04.2007 JP 2007098969**

(43) Date of publication of application:
**08.10.2008 Bulletin 2008/41**

(73) Proprietor: **ITEA Inc.**
**2-4, Yushima 2-chome**
**Bunkyo-ku, Tokyo (JP)**

(72) Inventors:
• **Miyazawa, Hiroshi**
**Tokyo (JP)**
• **Fujimura, Takashi**
**Chuo-ku**
**Chiba (JP)**
• **Sakaguchi, Masahiro**
**Tokyo (JP)**
• **Shira, Hideharu**
**Tokyo (JP)**

(74) Representative: **Beacham, Annabel Rose**
**Dehns**
**St Bride's House**
**10 Salisbury Square**
**London**
**EC4Y 8JD (GB)**

(56) References cited:
**US-A- 5 449 669**

• **YU CHIA-JUNG ET AL: "Proteomics and immunological analysis of a novel shrimp allergen, Pen m 2." JOURNAL OF IMMUNOLOGY, vol. 170, no. 1, 1 January 2003 (2003-01-01), pages 445-453, XP002487131 ISSN: 0022-1767**
• **MIYAZAWA ET AL: "Identification of the first major allergen of a squid (Todarodes pacificus)" JOURNAL OF ALLERGY AND CLINICAL IMMUNOLOGY, MOSBY - YEARLY BOOK, INC, US, vol. 98, no. 5, 1 November 1996 (1996-11-01), pages 948-953, XP005751565 ISSN: 0091-6749**
• **DUHAMEL R C ET AL: "REEXAMINATION OF THE AMINO-ACID COMPOSITIONS OF PROTEINS FROM PENAEID SHRIMP FAILURE TO FIND UNCOMMONLY HIGH TRYPTOPHAN LEVELS" COMPARATIVE BIOCHEMISTRY AND PHYSIOLOGY B, vol. 73, no. 2, 1982, pages 301-304, XP009102640 ISSN: 0305-0491**
• **BRAUER JOSAFAT MARINA EZQUERRA ET AL: "Effect of dietary protein on muscle collagen, collagenase and shear force of farmed white shrimp (Litopenaeus vannamei)." EUROPEAN FOOD RESEARCH AND TECHNOLOGY, vol. 217, no. 4, October 2003 (2003-10), pages 277-280, XP002487132 ISSN: 1438-2377**
• **AN H ET AL: "DEVELOPMENT OF MONOCLONAL ANTIBODY FOR ROCK SHRIMP IDENTIFICATION USING ELISA" JOURNAL OF AGRICULTURAL AND FOOD CHEMISTRY, vol. 38, no. 11, 1990, pages 2094-2100, XP002487133 ISSN: 0021-8561**
• **HAMADA YUKI ET AL: "Reactivity of IgE in fish-allergic patients to fish muscle collagen." ALLERGOLOGY INTERNATIONAL, vol. 52, no. 3, September 2003 (2003-09), pages 139-147, XP009102642 ISSN: 1323-8930**

**Description**

BACKGROUND OF THE INVENTION

1. Field of the Invention

The present invention relates to a method of detecting contamination by a shrimp allergen, use of an agent for detecting contamination by a shrimp allergen, a method of measuring sensitivity to a shrimp allergen, and use of an agent for measuring sensitivity to a shrimp allergen *in vitro*.

2. Description of the Related Art

Major foods causing food allergies in Japan include egg, milk, soybean, shrimp/crab, fish, etc. The incidence of allergies to each of these foods varies depending on the ages of patients, and adults have the highest incidence of developing allergies caused by shellfish such as shrimp and crab (see Yoneyama K, Ono A. Study of food allergy among university students in Japan. Allergology International 2002; 51: 205-208, for example).
The allergy caused by shrimp induces an immediate type reaction, and oral mucosal symptoms (such as labial numbness and oedema), skin symptoms (such as hives), gastrointestinal symptoms (such as nausea, vomiting, and diarrhea), and respiratory symptoms (such as asthma and breathlessness) may appear immediately after or within 30 minutes of ingestion of (or contact with) shrimp. Sometimes, severe systemic anaphylactic shock may be caused (see Shin Nakamura, Youji Iikura, Latest food allergy. Nagaishoten 2004; 241-244, for examples).
Conventionally, shrimp tropomyosin (TMS) and shrimp arginine kinase (AK) have been identified as materials causing shrimp allergy (see Daul CB, Slattery M, Reese G, et al : Identification of the major brown shrimp (Penaeus aztecus) allergen as the muscle protein tropomyosin. Int Arch Allergy Immunol 1994; 105: 49- 55 and Yu CJ, Lin YF, Chiang BL, Chow LP. Proteomics and immunological analysis of a novel shrimp allergen, Pen m 2. J Immunol. 2003; 170 445- 453, for example) .
Meanwhile, a recent report has revealed that fish collagen is an allergen for patients with fish allergy (see Hamada Y, Nagasima Y, Shiomi K, et al. Reactivity of IgE in fish- allergic patients to fish muscle collagen. Allergology International 2003; 52; 139- 147 and Sakaguchi M, Toda M, Ebihara T, et al. IgE antibody to fish gelatin (type I collagen) in patients with fish allergy. J Allergy Clin Immunol. 2000; 106: 579- 584, for example) .

SUMMARY OF THE INVENTION

Many patients with shrimp allergy have IgE antibodies against both or either of shrimp tropomyosin (TMS) and shrimp arginine kinase (AK). However, some patients have no antibodies against those allergens, and other unidentified allergens are considered to play important roles in causing the allergy.
Therefore, an object of the present invention is to provide a method of detecting contamination by a shrimp allergen, use of an agent for detecting contamination by a shrimp allergen, a method of measuring sensitivity to a shrimp allergen, and use of an agent for measuring sensitivity to a shrimp allergen *in vitro.*
The inventors of the present invention have made extensive studied to achieve the above-mentioned object, and as a result, the inventors have discovered that a collagen contained in the tail meat (muscle) of a shrimp is an allergen, thus accomplished the present invention.
That is, the present invention is as follows:

(1) a method of detecting contamination by a shrimp allergen in a sample including: preparing an extract from the sample; and detecting the presence or absence of one of shrimp collagen and a polypeptide fragment thereof in the extract;
(2) a method of detecting contamination by a shrimp allergen according to Item (1), in which the sample is one of a food, drink, and a material thereof;
(3) a method of detecting contamination by a shrimp allergen according to Item (1) or (2), in which the shrimp collagen is derived from *Penaeus japonicus* or *Pandalus platyceros*;
(4) a method of detecting contamination by a shrimp allergen according to any one of Items (1) to (3), in which the method of detecting the presence or absence of one of shrimp collagen and a polypeptide fragment thereof in the extract is immunological detection means using an anti-shrimp allergen antibody, the anti-shrimp allergen antibody recognizing one of the shrimp collagen and the polypeptide fragment thereof; the anti-shrimp allergen antibody is preferably one of an antiserum, an IgG fraction, a polyclonal antibody, and a monoclonal antibody;
(5) use of an agent including an anti-shrimp allergen antibody recognizing one of the shrimp collagen and the polypeptide fragment thereof, for detecting contamination by a shrimp allergen in a sample;

(6) use according to Item (5), in which the anti-shrimp allergen antibody is contained in a solution;

(7) use according to Item (5), in which the anti-shrimp allergen antibody is supported in a support matrix;

(8) a method of measuring sensitivity to a shrimp allergen including: allowing a biological sample of one of a subject and a preparation thereof to react with a shrimp allergen including one of an isolated shrimp collagen and a polypeptide fragment thereof, in which the isolated shrimp collagen preferably has a molecular weight of 110 to 140 kDa determined by SDS-polyacrylamide gel electrophoresis analysis, and/or is derived from *Penaeus japonicus* or *Pandalus platyceros*; and measuring an amount of an IgE antibody binding to the shrimp allergen among IgE antibodies contained in one of the biological sample and preparation thereof;

(9) use of an agent including a shrimp allergen including one of an isolated shrimp collagen and a polypeptide fragment thereof, in which the isolated shrimp collagen preferably has a molecular weight of 110 to 140 kDa determined by SDS-polyacrylamide gel electrophoresis analysis, and/or is derived from *Penaeus japonicus* or *Pandalus platyceros*, for measuring sensitivity of a subject to a shrimp allergen *in vitro*;

(10) use of an agent for measuring sensitivity to a shrimp allergen according to Item (9), in which the shrimp allergen is contained in a solution;

(11) use of an agent for measuring sensitivity to a shrimp allergen according to Item (10), in which the shrimp allergen is supported in a support matrix; and

(12) use of an agent for measuring sensitivity to a shrimp allergen according to any one of Items (10) to (11), the agent further including a labeled anti-IgE antibody.

According to the present invention, there is provided a method of detecting contamination by a shrimp allergen, use of an agent for detecting contamination by a shrimp allergen, a method of measuring sensitivity to a shrimp allergen, and use of an agent for measuring sensitivity to a shrimp allergen *in vitro*.

BRIEF DESCRIPTION OF THE DRAWINGS

In the accompanying drawings:

Fig. 1 is a photograph of a purified *Penaeus japonicus* collagen product obtained in Production Example 1, which was subjected to SDS-polyacrylamide gel electrophoresis and Coomassie staining;

Fig. 2 is a graph showing results of staining with Sirius-red, which is a dye specifically binding to the helix structure of an undenatured collagen;

Fig. 3 its a graph showing results of comparison of the ultraviolet absorption characteristics of *Penaeus japonicus* collagen obtained in Production Example 1 with those of bovine collagen and BSA (bovine serum albumen); and

Fig. 4 is a graph showing results of inhibition ELISA using shrimp, mackerel, and bovine collagens as inhibitors.

DETAILED DESCRIPTION OF THE PREFERRED EMBODIMENTS

The present invention has been made by newly discovering that a collagen contained in the tail meat (muscle) of a shrimp is an allergen. That is, the shrimp allergen of the present invention includes shrimp collagen or a polypeptide fragment thereof.

A shrimp collagen to be used in the present invention can be obtained by known techniques such as the method of Ebihara and Irie (Tetsuya Ebihara and Shinkichi Irie: Fish and invertebrate collagen; Method for studying cytology matrix (I), published by Collagen Technique Workshop 1998: 52-57). That is, the collagen can be obtained from the tail meat (muscle) of a shrimp as a material by step-wise treatments such as homogenization, washing with a washing solution, delipidation with ethanol, digestion/extraction with pepsin, salting out with NaCl, and dialysis after solubilization with an acetic acid solution.

Collagens are present in various organisms, and their structures and properties are similar. Basically, a collagen is composed of three polypeptide chains with molecular weights of about 100, 000, and the polypeptide chains of an undenatured collagen have helical structures. If a collagen is developed on a gel by SDS- polyacrylamide gel electrophoresis, the polypeptide chains separate into a monomer including one polypeptide chain ($\alpha$- chain), a dimer including two polypeptide chains ($\beta$- chain), and a trimer including three polypeptide chains ($\gamma$- chain) . However, the extractabilities from material tissues of the molecular species (monomer ($\alpha$- chain), dimer ($\beta$- chain), and trimer (y- chain) ) vary significantly depending on the species of animal or tissues being used.

A shrimp collagen to be used in the present invention can be easily obtained as a purified collagen fraction by repeating a step of digestion with pepsin twice at the time of extraction, from shrimp muscle tissues. The resultant purified collagen fraction contains a monomer ($\alpha$-chain) of a collagen purified enough to appear as an almost single band in SDS-polyacrylamide gel electrophoresis-Coomassie staining analysis.

The shrimp collagen to be used in the present invention can be appropriately produced by a method other than the above-mentioned method. In addition, the shrimp collagen may be appropriately concentrated and purified by known techniques.

The species of shrimp to be used as a source material is not particularly limited, and preferable examples thereof include edible shrimps such as *Penaeus japonicus* and *Pandalus platyceros*.

The shrimp allergen to be used in the present invention may include a polypeptide fragment of shrimp collagen as long as the fragment has immunological properties of the shrimp collagen, such as allergenicity, antibody productivity, and antibody binding capacity. That is, for example, a general epitope that is a local structure for determining specificity of an antigen-antibody reaction has an amino acid length of about 10 to several tens of amino acids. Therefore, shrimp collagen is considered to contain a partial polypeptide fragment having such immunological properties.

The term "immunological" as used herein is used as a synonym for a term generally used in the art, and includes not only one using immunity to humans but also one using immunity to other animals such as a rabbit, goat, sheep, monkey, bovine, chicken, guinea pig, rat, and mouse.

The shrimp allergen to be used in the present invention may reversibly or irreversibly bind to another substance before use as long as it maintains its immunological properties. For example, the allergen may bind to, for example, resins, glasses, plastics, metals, dye molecules, radioactive substances, fluorescent substances, biopolymers, and organic material molecules before use. The form of a material to bind to the allergen is not particularly limited, and the material may be in the form of, for example, a gel, a nanoparticle, a colloid, a bead, a porous material, and a plate.

The shrimp allergen can be used as follows.

For example, an extract was prepared from a sample, and the shrimp allergen of the present invention (shrimp collagen or a polypeptide fragment thereof) was used as a material index to detect the presence or absence of a shrimp allergen (shrimp collagen or a polypeptide fragment thereof) in the extract. Examples of the sample include foods and drinks, and materials thereof.

This method enables easy checking for contamination by a shrimp allergen in a food or drink for manufacturers or managers thereof, and if the results are indicated on a package or the like, buyers can be made aware of the presence of the allergen. As a result, it is possible to prevent consumers who have developed or may develop shrimp allergy from ingesting the food or drink.

In addition, collagen is known to maintain its antigenicity even if it is melted into gelatin. Therefore, in the case where the shrimp allergen of the present invention (a shrimp collagen or a polypeptide fragment) is used as a material index, the antigenicity of the shrimp allergen in a heat-treated food or drink is considered to be maintained and can be easily detected by the immunological detection means described below.

Moreover, the shrimp allergen can be used as follows.

For example, a shrimp allergen of the present invention is allowed to react with a biological sample from a subject, or a preparation thereof, and an amount of an IgE antibody capable of binding to the shrimp allergen among IgE antibodies contained in the biological sample or preparation thereof is measured. This method can measure sensitivity of the subject to the shrimp collagen or measure the conditions of the allergy symptom caused by shrimp collagen.

Examples of the biological sample from a subject, or a preparation thereof, include blood, serum, and plasma.

The amount of the IgE antibody capable of binding to the shrimp allergen can be measured by a method including: immobilizing the shrimp allergen of the present invention on a microplate or beads; separating an IgE antibody capable of binding to the solid phase via the shrimp allergen of the present invention among IgE antibodies contained in the biological sample from a subject, or the preparation thereof, from antibodies that cannot bind thereto and stay in the solution by washing or centrifugation; and measuring the amount of the antibody using an antibody against the IgE antibody. Examples of the antibody against the IgE antibody include a commercially available product such as anti-human IgE-β-galactosidase-labeled antibody.

In order to perform the above-mentioned measurement method easily and rapidly, an agent for measuring sensitivity to a shrimp allergen including a shrimp allergen of the present invention may be used. Moreover, the agent for measuring sensitivity to a shrimp allergen may further include an optional agent, as needed, in addition to the shrimp allergen of the present invention. Examples of the optional agent include a labeled anti-IgE antibody such as the above-mentioned anti-human IgE-β-galactosidase-labeled antibody.

The anti-shrimp allergen antibody used in the present invention is an anti-shrimp allergen antibody capable of recognizing a shrimp allergen of the present invention, and specific examples thereof include anti-shrimp allergen antibodies such as antiserums, IgG fractions, polyclonal antibodies, and monoclonal antibodies. The antibody can be prepared by any technique known in the art. For example, a shrimp allergen of the present invention can be administered to an animal to be immunized, and the antiserum then collected from the animals. Examples of suitable animals include a rabbit, goat, sheep, monkey, bovine, chicken, guinea pig, rat, and mouse.

The anti-shrimp allergen antibody used in the present invention may be reversibly or irreversibly bound to another substance before use as long as it maintains its immunological properties. For example, the antibody may bind to, for example, resins, glasses, plastics, metals, dye molecules, radioactive substances, fluorescent substances, biopolymers, and organic material molecules before use. The form of a material to be bound to the antibody is not particularly limited, and the material may be in the form of, for example, a gel, a nanoparticle, a colloid, a bead, a porous material, and a plate.

The anti-shrimp allergen antibody can be used as follows.

For example, in the above-mentioned method of detecting the presence or absence of a shrimp allergen in a sample, immunological detection means using the anti-shrimp allergen antibody of the present invention may be used as means for detecting the presence or absence of shrimp collagen or a polypeptide thereof.

Various methods such as ELISA and immunochromatography are widely used as immunological detection means, and a person skilled in the art can easily use an appropriate known technique.

In order to perform the above-mentioned detecting method easily and rapidly, an agent for detecting contamination by a shrimp allergen including an anti-shrimp allergen antibody of the present invention may be used. Moreover, the agent for detecting contamination by a shrimp allergen may further include an optional agent, as needed, in addition to the anti-shrimp allergen antibody of the present invention. Examples of the optional agent include an anti-IgG secondary antibody such as anti-IgG-FC fragment antibody capable of recognizing an anti-shrimp allergen antibody.

Examples

Hereinafter, the present invention will be described specifically by way of examples, but they are not intended to limit the scope of the present invention.

<Production Example 1> Purification of *Penaeus japonicus* collagen

In accordance with the method of Ebihara and Irie (Tetsuya Ebihara and Shinkichi Irie: Fish and invertebrate collagen; Method for studying cytology matrix (I), published by Collagen Technique Workshop, 1998; 52-57), collagen was obtained by purifying the tail meat of *Penaeus japonicus* according to the following procedure.

The tail meat was placed in a washing solution (0.1 $\mu$M N-ethylmaleimide, 10 $\mu$M PMSF, 1 mM EDTA, 25 mM $Na_2HPO_4$; prepared before use) cooled with ice and homogenized using a glass homogenizer. The solution was centrifuged at 10,000 rpm (15,000 $\times$ g) for 10 minutes at 4°C (the following centrifugation processes were performed under those centrifugation conditions unless otherwise specified), and further washing solution was added to the precipitates, followed by stirring for 3 to 10 hours. The mixture was centrifuged again, and further washing solution was added to the precipitates, followed by stirring. This washing step was repeated 5 to 7 times. The protein content in the supernatant was confirmed to reach 10 $\mu$g/ml or less, and an about 10-fold amount of cold ethanol was added to the precipitates, followed by stirring for 3 hours. This step was repeated several times until the supernatant became clear. To the resultant delipidated precipitates was added an about 10-fold amount of 1 mg/ml pepsin-0.5 M acetic acid (extraction solution), and the mixture was stirred overnight. The resultant mixture was centrifuged, and the supernatant was collected. In addition, the extraction solution was again added to the precipitates obtained by centrifugation to perform extraction in the same way above. The supernatants obtained by centrifugation were combined, and NaCl was added thereto so that the final concentration was 2 M, followed by stirring overnight. The mixture was centrifuged, and an about 10-fold amount of 0.05 M acetic acid was added to the resultant precipitates to dissolve them. The solution was centrifuged again, and the supernatant was placed in a dialysis membrane (manufactured by PIERCE, Rockford, IL). The supernatant was dialyzed against 0.005 M Tris-HCl (pH 7.5), and the dialysis step was repeated until the pH value of the external solution was stabilized at 7.5. After dialysis, the mixture was centrifuged, and an about 10-fold amount of 0.05 M acetic acid was added to the precipitates to dissolve them, followed by centrifugation again. The supernatant was collected and dialyzed against 0.005 M acetic acid overnight, followed by centrifugation for 20 minutes, to thereby yield the supernatant (purified *Penaeus japonicus* collagen product).

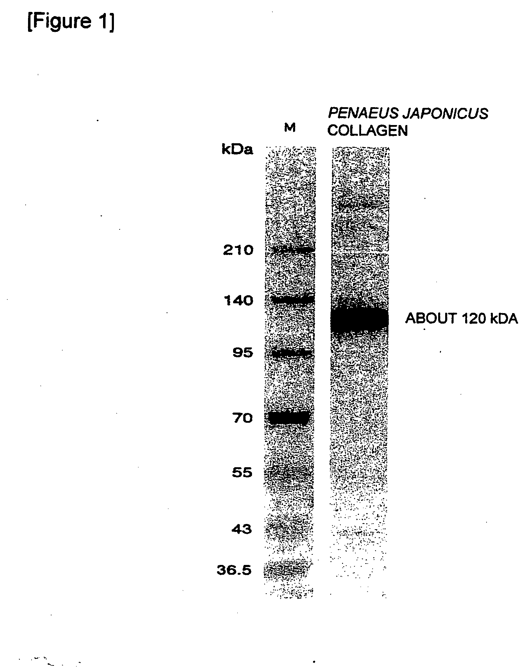

The resultant supernatant was subjected to SDS-polyacrylamide gel electrophoresis, followed by Coomassie staining. The results are shown in Fig. 1.

Collagens are present in various organisms, and their structures and properties are similar. Basically, a collagen is composed of three polypeptide chains with molecular weights of about 100, 000, and the polypeptide chains of an undenatured collagen have helix structures. If a collagen is developed on a gel by SDS- polyacrylamide gel electrophoresis, the polypeptide chains separate into a monomer including one polypeptide chain ($\alpha$- chain), a dimer including two polypeptide chains ($\beta$- chain), and a trimer including three polypeptide chains ($\gamma$- chain). As shown in Fig. 1, comparison with a molecular weight marker revealed that the resultant supernatant was confirmed as a major band corresponding to about 120 kDa, which was considered to be a collagen monomer ($\alpha$- chain). Meanwhile, faint bands corresponding to a collagen dimer ($\beta$- chain) and a collagen trimer ($\gamma$- chain) were also detected. In addition, another faint band was detected at about 42 kDa.

<Test Example 1> Confirmation of shrimp allergen contaminant

Many patients with shrimp allergy have specific IgE antibodies against both or either of shrimp tropomyosin (TMS) and shrimp arginine kinase (AK).

Therefore, an anti- shrimp tropomyosin antibody (derived from mouse) and an anti- shrimp arginine kinase antibody (derived from mouse) were used to examine whether shrimp tropomyosin and shrimp arginine kinase were present as contaminants in the purified *Penaeus japonicus* collagen product obtained in Production Example 1 according to the following procedures. Those antibodies were prepared as follows. That is, the tail meat of *Penaeus chinensis* (belonging to the genus *Penaeus*) was boiled, and the resultant extract was subjected to salting out with ammonium sulfate and purified using an anion- exchange column and a hydroxyapatite column to isolate shrimp tropomyosin. The purified tropomyosin was used in the immunization of a mouse together with alum (adjuvant), and an anti- shrimp tropomyosin antibody (mouse antiserum) was separated in accordance with a conventional method. On the other hand, a homogenate of the tail meat of *Penaeus japonicus* was prepared, and it was subjected to salting out with ammonium sulfate and purified using a nickel chelate column to isolate arginine kinase. The purified arginine kinase was used in the immunization of a mouse together with alum (adjuvant), and an anti- shrimp arginine kinase antibody (mouse antiserum) was obtained in accordance with a conventional method.

The concentration of the purified *Penaeus japonicus* collagen product obtained in Production Example 1 was adjusted to 10 μg/ml with 0.05 M carbonic acid- bicarbonic acid buffer (pH 9.6), and the solution was added to a 96- well flat- bottomed microplate at 100 μl (about 1 μg) / well. The microplate was allowed to stand at 4°C overnight to immobilize the collagen. The microplate was washed with PBST three times, and the above- mentioned major shrimp allergen antibodies (mouse antiserums) (anti- TMS and anti- AK antibodies, each of them was diluted at 25, 000- fold) were allowed to react at room temperature for 60 minutes. The microplate was washed, and alkaline phosphatase (Alp)- labeled anti- mouse IgG antibody (diluted at 2, 000- fold) was allowed to react at room temperature for 60 minutes. The microplate was washed, and 1 mg/ml p- nitro phenyl phosphate was allowed to react at room temperature for 15 minutes. The reaction was stopped by adding 0.2 M EDTA, and absorbances at 405 nm were measured. Meanwhile, as positive controls for the two antibodies, shrimp tropomyosin (TMS) and shrimp arginine kinase (AK) were immobilized on a 96- well flat- bottomed microplate at about 0.1 μg/ well, and the absorbances were measured in the same way as above. The results are shown in Table 1.

[Table 1]

|  | Immobilized antigen | | |
|---|---|---|---|
| Major shrimp allergen antibody | Blank | *Penaeus japonicus* collagen* | Major shrimp allergen# |
| Anti-tropomyosin (TMS) | 0.08 | 0.08 | >2.0 (TMS) |
| Anti-arginine kinase (AK) | 0.079 | 0.08 | >2.0 (AK) |
| * *Penaeus japonicus* collagen was immobilized at 10 μg/ml, # Major shrimp allergens were immobilized at 1 μg/ml: antibodies against two major shrimp allergens (tropomyosin (TMS) and arginine kinase (AK)) (mouse polyclonal antibodies) were allowed to react at 1:25,000 and were detected with anti-mouse IgG AI-p labeled-antibody and pNPP. | | | |

As shown in Table 1, the purified *Penaeus japonicus* collagen product obtained in Production Example 1 did not react with the anti-shrimp tropomyosin antibody (mouse antiserum) and anti-shrimp arginine kinase antibody (mouse antiserum) at all, and the amounts (absorbances) of the two antibodies that reacted with a shrimp collagen were found to be equal to the that of a blank.

Those results and the above-mentioned results of SDS-polyacrylamide gel electrophoresis revealed that the purified *Penaeus japonicus* collagen product obtained in Production Example 1 did not contain shrimp tropomyosin (TMS) or shrimp arginine kinase (AK) and was purified enough to evaluate allergenicity of the shrimp collagen.

<Test Example 2> Identification of *Penaeus japonicus* collagen

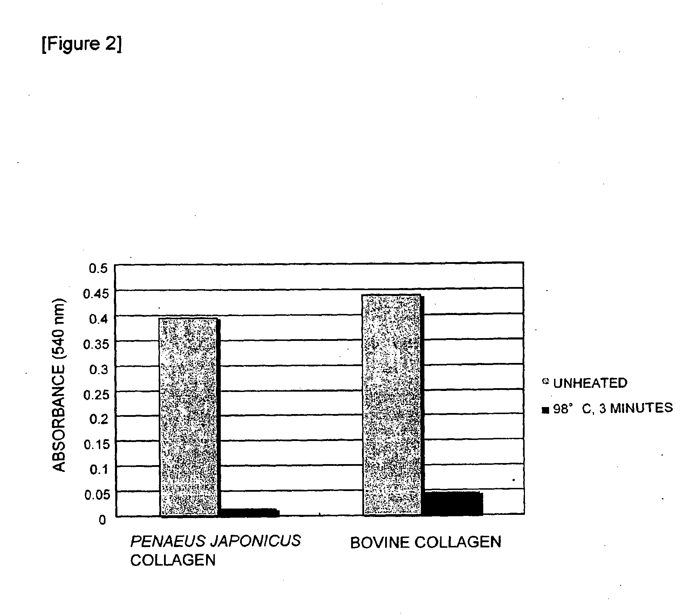

The protein obtained in Production Example 1 was stained with Sirius-red, which is a dye which specifically binds to the helix structure of an undenatured collagen, to  confirm the protein to be a collagen. Specifically, for the purified *Penaeus japonicus* collagen product obtained in Production Example 1, a collagen identification kit, "Sircol Collagen Assay kit" (product name, manufactured by Biodye Science, Northern Ireland, UK) was used to determine the degree of staining with Sirius-red. On the other hand, for a product obtained by heating (denaturing) the sample, the degree was determined in the same way as above. The results are shown in Fig. 2.

As shown in Fig. 2, the unheated (undenatured) sample could be stained with the dye (Sirius- red), while the heated (denatured) sample was found to have significantly reduced dye- affinity. The results were analogous to those using bovine collagen.

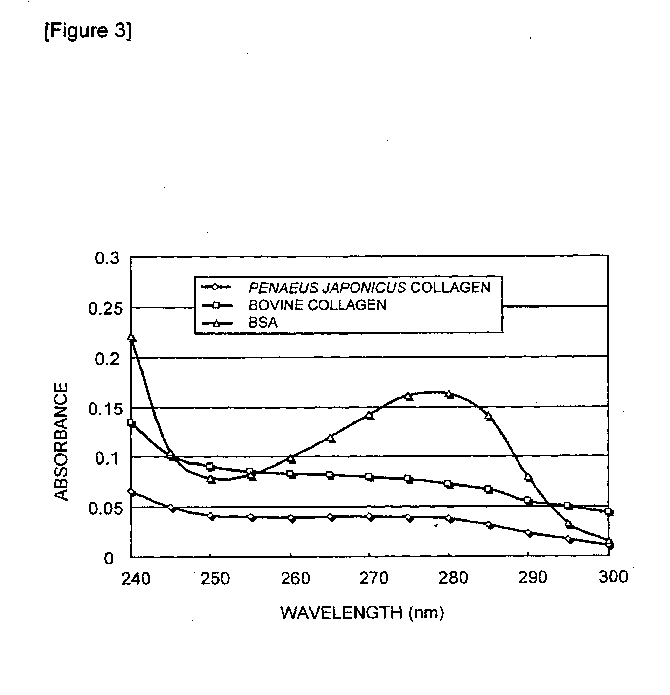

The collagen has an extremely unique amino acid composition, and contains a high level of glycine, a low level of a hydrophobic amino acid, and hardly contains tyrosine, and therefore the absorbance at 280 nm is scarcely detected. Fig. 3 shows a comparison of the ultraviolet absorption characteristics of the protein (*Penaeus japonicus* collagen) obtained in Production Example 1 with those of bovine collagen and BSA (bovine serum albumin). Specifically, each of the concentrations of the purified *Penaeus japonicus* collagen product obtained in Production Example 1, bovine collagen, and BSA was adjusted to 1 mg/ml with purified water, and their ultraviolet absorption characteristics at wavelengths of 240 to 300 nm were compared.

As shown in Fig. 3, in the case of BSA, a clear peak was detected at 280 nm, while in the case of the purified *Penaeus japonicus* collagen product obtained in Production  Example 1 or bovine collagen, no peak was detected at 280 nm.

As a result, the protein obtained in Production Example 1 was identified as a collagen.

<Production Example 2> Purification of *Pandalus platyceros* collagen

In the same way as Production Example 1, a collagen was obtained by purifying the tail meat of *Pandalus platyceros.* As a result, *Pandalus platyceros* collagen having characteristics similar to those of *Penaeus japonicus* and having a molecular weight of about 120 kDa could be obtained.

<Test Example 3> Allergenicity of shrimp collagen

Indirect ELISA assay was performed according to the following procedure to examine whether the expression levels of IgE antibodies against a shrimp collagen were increased or not in the serums of patients with shrimp allergy (23 shrimp CAP- RAST positive subjects) . The patients with shrimp allergy had developed allergic symptoms such as labial oedema or hives due to ingestion of shrimp, and contact dermatitis (during cooking), and their serums were evaluated as positive by a commercially- available allergen IgE antibody test kit (CAP- R.AST) .

The concentration of the shrimp collagen obtained in Production Example 1 or 2 was adjusted to 10 $\mu$g/ml with 0.05 M carbonic acid- bicarbonic acid buffer (pH 9.6), and the shrimp collagen was immobilized on a 96- well flat-bottomed microplate at about 1 $\mu$g/ well. The microplate was washed with PBST (PBS, 0.05% Tween 20) three times, and the serums of the patients with shrimp allergy were diluted 5- fold with 1% BSA- PBST. The diluted samples were dispensed and allowed to react at room temperature for 3 hours. The microplate  was washed with PBST three times, and an anti- human IgE- $\beta$- galactosidase- labeled antibody ("CAP- RAST FEIA kit" (product name), manufactured by Phadia) was diluted 15- fold with 1% BSA- PBST, and the diluted samples were dispensed and allowed to react at 4°C overnight. The microplate was washed with PBST three times, and 0.3 mM 4- methylumbelliferyl- $\beta$- D- galactosidase was added and allowed to react in an incubator at 37°C for 90 minutes. Finally, 0.1 M glycine- NaOH (pH 10.2) was added to stop the reaction. Fluorescence units were measured using a microplate reader "Fluoroskan II" (product name, manufactured by Titertek, USA), and a sample having 100 FU or more was judged as antibody- positive.

The results are shown in Table 2.

[Table 2]

|  | Collagen IgE (>100 FU) | |
| --- | --- | --- |
| Collagen | Number of positive subjects/n | Positive ratio (%) |
| *Penaeus japonicus* | 8/23 | 35 |
| *Pandalus platyceros* | 5/23 | 22 |

As shown in Table 2, in the case of using *Penaeus japonicus* collagen as an antigen, 8 out of the 23 cases (35%) of patients with shrimp allergy were antibody-positive. On the other hand, in the case of using *Pandalus platyceros* collagen as an antigen, 5 out of the 23 cases (22%) of patients with shrimp allergy were antibody-positive.

The results revealed that the expression levels of IgE antibodies against the shrimp collagens were increased in the serums of some of the patients with shrimp allergy (23 shrimp CAP-RAST positive subjects). Therefore, it was suggested that shrimp collagen is one of the materials causing shrimp allergy symptoms.

<Test Example 4> Cross-reactivity to collagens derived from other animals.

Collagens are present in various organisms, and their structures and properties are similar. Meanwhile, Sakaguchi et al. have reported that the positive ratio of a fish gelatin IgE antibody was about 30% in patients with fish allergy (see Sakaguchi M, Toda M, Ebihara T, et al. IgE antibody to fish gelatin (type I collagen) in patients with fish allergy. J Allergy Clin Immunol. 2000; 106: 579-584).

Therefore, the cross- reactivity of the shrimp collagens shown in Test Example 3 were determined to demonstrate that allergenicity of the shrimp collagens was not caused by reactions with IgE antibodies induced by collagens that were derived from other animals and present in the serums of the patients with shrimp allergy. Specifically, inhibition ELISA was performed using mackerel and bovine collagens to determine specificity of collagen IgE antibodies present in the serums of the patients with shrimp allergy.

First, the concentration of the *Penaeus japonicus* collagen obtained in Production Example 1 was adjusted to 10 μg/ml with 0.05 M carbonic acid- bicarbonic acid buffer (pH 9.6), and the collagen was immobilized on a 96- well flat-bottomed microplate at about 1 μg/ well. Then, solutions obtained by serially diluting each of the *Penaeus japonicus* collagen obtained in Production Example 1 and mackerel and bovine collagens to 20 μg/ml, 4 μg/ml, and 0.8 μg/ml were added to other test tubes, and a collagen IgE- positive pooled serum (a mixture of the serums of the 3 positive subjects in Test Example 3) diluted 5- fold was mixed in an equal amount, followed by a reaction at 4°C overnight. The collagen- immobilized plate was washed with PBST three times, and the mixtures of the collagens and serums prepared in the test tubes were dispensed at 100 μl/ well, followed by a reaction at room temperature for 3 hours. The following procedures were performed in the same way as Test Example 3 to measure absorbances. The inhibition ratios were calculated from the following formula.

$$\cdot \text{Inhibition ratio (\%)} = (\text{FU in well containing no inhibitor} - \text{FU in well containing inhibitor}) \div \text{FU in well containing no inhibitor} \times 100$$

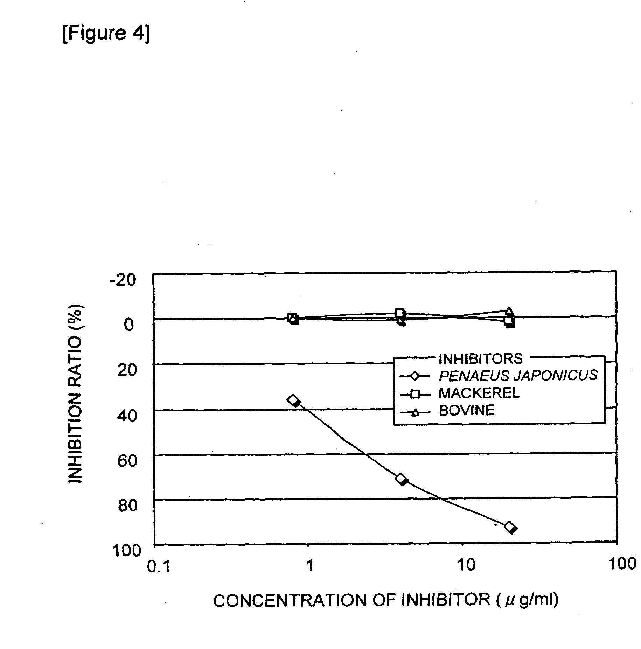

The results are shown in Fig. 4. As shown in Fig. 4, in the case of using the shrimp collagen as an inhibitor, the reaction of the immobilized *Penaeus japonicus* collagen with the serum were inhibited in a concentration-dependent manner. On the other hand, in the cases of using the mackerel and bovine collagens as inhibitors, the reaction was not inhibited at all.

The results revealed that the shrimp collagen is an allergen with low cross-reactivity to other collagens. Therefore, it was considered that the allergenicity of the shrimp collagen shown in Test Example 3 was caused not by a reaction with an IgE antibody induced by collagens that were derived from other animals and present in the serums of the patients with shrimp allergy, but by an increase in the expression level of an IgE antibody specific to shrimp collagen.

<Example 1> Detection of carrier of shrimp collagen-specific IgE antibody

Among 175 normal adult subjects, carriers of IgE antibodies against shrimp or mackerel collagen were detected. Specifically, serums were collected from the subjects, and positive subjects were detected in the same way as Test Example 3. The results are shown in Table 3.

[Table 3]

| Serum No. | Collagen IgE (FU) | | Symptom | Causative food |
|---|---|---|---|---|
| | *Penaeus japonicus* | Mackerel | | |
| 1 | 833 | 2350 | Hives | Shrimp, Crab |
| 2 | 793 | - | Hives | Shrimp |
| 3 | 106 | - | Labial edema | Shrimp, Crab |
| 4 | - | 3849 | Hives | Fish cake (Kamaboko) |
| 5 | - | 328 | Not clear | Not clear |
| 6 | - | 163 | Anaphylaxis | Buck wheat noodle (Soba) |

(continued)

| | Collagen IgE (FU) | | | |
|---|---|---|---|---|
| Serum No. | *Penaeus japonicus* | Mackerel | Symptom | Causative food |
| Number of positive subjects (≥100 FU) | 3 | 4 | | |
| Number of negative subjects | 172 | 171 | | |
| Positive ratio (%) | 1.7 | 2.3 | | |

As shown in Table 3, 3 subjects (1.7%) were evaluated as IgE antibody-positive against *Penaeus japonicus* collagen, while 4 subjects (2.3%) were evaluated as IgE antibody-positive against mackerel collagen. The antibody levels in the case of shrimp were not correlated with those in the case of mackerel, and only one subject was found to have IgE antibodies against both shrimp and mackerel. In addition, the 3 subjects evaluated to have the IgE antibody against shrimp collagen had each developed allergic symptoms such as labial oedema or hives due to ingestion of shrimp or crab.

As shown above, the shrimp allergic subjects were correlated with the subjects evaluated as carriers of an IgE antibody against shrimp collagen, and therefore, it was verified that a shrimp collagen as allergens and an anti-shrimp allergen antibody prepared by using the collagen could be applied to measurement of shrimp allergy or the like.

**Claims**

1. A method of detecting contamination of a sample by a shrimp allergen, comprising:

   preparing an extract from the sample; and
   detecting the presence or absence of one of a shrimp collagen and a polypeptide fragment thereof in the extract.

2. A method of detecting contamination by a shrimp allergen according to Claim 1, wherein the sample is one of a food, a drink, and an ingredient thereof.

3. A method of detecting contamination by a shrimp allergen according to Claim 1 or 2, wherein the shrimp collagen is derived from *Penaeus japonicus* or *Pandalus platyceros.*

4. A method of detecting contamination by a shrimp allergen according to any one of Claims 1 to 3, wherein the method of detecting the presence or absence of one of the shrimp collagen and the polypeptide fragment thereof in the extract is an immunological detection means using an anti-shrimp allergen antibody, the anti-shrimp allergen antibody recognizing one of the shrimp collagen and the polypeptide fragment thereof.

5. Use of an agent comprising an anti--shrimp allergen antibody recognizing one of the shrimp collagen and the polypeptide fragment thereof, for detecting contamination of a sample by a shrimp allergen.

6. Use according to Claim 5, wherein the anti-shrimp allergen antibody is contained in a solution.

7. Use according to Claim 5, wherein the anti-shrimp allergen antibody is supported in a support matrix.

8. Use according to any of Claims 5 to 7, wherein the sample is one of a food, a drink, and an ingredient thereof.

9. A method of measuring sensitivity to a shrimp allergen comprising:

   allowing a biological sample from a subject, or a preparation thereof, to react with a shrimp allergen comprising one of an isolated shrimp collagen and a polypeptide fragment thereof; and
   measuring an amount of an IgE antibody binding to the shrimp allergen among IgE antibodies contained in one of the biological sample and the preparation thereof.

10. A method according to Claim 9, wherein the isolated shrimp collagen has a molecular weight of 110 to 140 kDa determined by SDS-polyacrylamide gel electrophoresis analysis.

**11.** A method according to Claim 9 or Claim 10, wherein the isolated shrimp collagen is derived from *Penaeus japonicas* or *Pandalus platyceros.*

**12.** Use of an agent comprising a shrimp allergen comprising one of an isolated shrimp collagen and a polypeptide fragment thereof, for measuring sensitivity of a subject to a shrimp allergen *in vitro.*

**13.** Use according to Claim 12, wherein the isolated shrimp collagen has a molecular weight of 110 to 140 kDa determined by SDS-polyacrylamide gel electrophoresis analysis.

**14.** Use according to Claim 12 or Claim 13, wherein the isolated shrimp collagen is derived from *Penaeus japonicus* or *Pandalus platyceros.*

**15.** Use according to any one of Claims 12 to 14, wherein the agent further comprises a labelled anti-IgE antibody.

**Patentansprüche**

**1.** Verfahren zum Nachweis der Kontamination einer Probe durch ein Garnelenallergen, umfassend:

Herstellen eines Extrakts aus der Probe; und
Nachweis des Vorhandenseins oder der Abwesenheit eines Garnelenkollagens oder eines Polypeptidfragments davon in dem Extrakt.

**2.** Verfahren zum Nachweis der Kontamination durch ein Garnelenallergen nach Anspruch 1, wobei die Probe ein Nahrungsmittel, ein Getränk oder ein Inhaltsstoff davon ist.

**3.** Verfahren zum Nachweis der Kontamination durch ein Garnelenallergen nach Anspruch 1 oder 2, wobei das Garnelenkollagen aus *Penaeus japonicus* oder *Pandalus platyceros* stammt.

**4.** Verfahren zum Nachweis der Kontamination durch ein Garnelenallergen nach einem der Ansprüche 1 bis 3, wobei das Verfahren zum Nachweis des Vorhandenseins oder der Abwesenheit des Garnelenkollagens oder des Polypeptidfragments davon in dem Extrakt ein immunologisches Nachweismittel ist, bei welchem ein gegen das Garnelenallergen gerichteter Antikörper verwendet wird, wobei der gegen das Garnelenallergen gerichtete Antikörper das Garnelenkollagen oder das Polypeptidfragment davon erkennt.

**5.** Verwendung eines Agens, das einen gegen das Garnelenallergen gerichteten Antikörper umfasst, der das Garnelenkollagen oder das Polypeptidfragment davon erkennt, zum Nachweis der Kontamination einer Probe durch ein Garnelenallergen.

**6.** Verwendung nach Anspruch 5, wobei der gegen das Garnelenallergen gerichtete Antikörper in einer Lösung enthalten ist.

**7.** Verwendung nach Anspruch 5, wobei der gegen das Garnelenallergen gerichtete Antikörper in einer Trägermatrix gehalten wird.

**8.** Verwendung nach einem der Ansprüche 5 bis 7, wobei die Probe ein Lebensmittel, ein Getränk oder ein Inhaltsstoff davon ist.

**9.** Verfahren zur Bestimmung der Empfindlichkeit gegenüber einem Garnelenallergen, umfassend
Reagierenlassen einer aus einem Subjekt entnommenen biologischen Probe oder einer Präparation davon mit einem Garnelenallergen, das ein isoliertes Garnelenkollagen oder ein Polypeptidfragment davon enthält; und
Bestimmen einer Menge eines IgE-Antikörpers, der sich an das Garnelenallergen bindet und sich unter den IgE-Antikörpern befindet, die in der biologischen Probe oder der Präparation davon enthalten sind.

**10.** Verfahren nach Anspruch 9, wobei das isolierte Garnelenkollagen ein Molekulargewicht von 110 bis 140 kDa aufweist, wie durch Elektrophoreseanalyse mit SDS-Polyacrylamidgel bestimmt wurde.

**11.** Verfahren nach Anspruch 9 oder Anspruch 10, wobei das isolierte Garnelenkollagen aus *Penaeus japonicus* oder *Pandalus platyceros* stammt.

12. Verwendung eines Agens, umfassend ein Garnelenallergen, das ein isoliertes Garnelenkollagen oder ein Polypeptidfragment davon umfasst, zur Bestimmung der Empfindlichkeit eines Subjekts gegenüber einem Garnelenallergen *in vitro*.

13. Verwendung nach Anspruch 12, wobei das isolierte Garnelenkollagen ein Molekulargewicht von 110 bis 140 kDa aufweist, wie durch Elektrophoreseanalyse mit SDS-Polyacrylamidgel bestimmt wurde.

14. Verwendung nach Anspruch 12 oder Anspruch 13, wobei das isolierte Garnelenkollagen aus *Penaeus japonicus* oder *Pandalus platyceros* stammt.

15. Verwendung nach einem der Ansprüche 12 bis 14, wobei das Agens ferner einen markierten anti-IgE-Antikörper umfasst.

**Revendications**

1. Procédé de détection de contamination d'un échantillon par un allergène de crevette, comprenant les étapes consistant à :

    préparer un extrait à partir de l'échantillon ; et
    détecter la présence ou l'absence d'un collagène de crevette ou d'un fragment polypeptidique de celui-ci dans l'extrait.

2. Procédé de détection de contamination par un allergène de crevette selon la revendication 1, dans lequel l'échantillon est choisi parmi un aliment, une boisson et un de leurs ingrédients.

3. Procédé de détection de contamination par un allergène de crevette selon la revendication 1 ou la revendication 2, dans lequel le collagène de crevette est dérivé du *Penaeus japonicus* ou du *Pandalus platyceros.*

4. Procédé de détection de contamination par un allergène de crevette selon l'une quelconque des revendications 1 à 3, dans lequel le procédé de détection de la présence ou de l'absence du collagène de crevette ou du fragment polypeptidique de celui-ci dans l'extrait est un moyen de détection immunologique utilisant un anticorps allergène anti-crevette, l'anticorps allergène anti-crevette reconnaissant le collagène de crevette ou le fragment polypeptidique de celui-ci.

5. Utilisation d'un agent comprenant un anticorps allergène anti-crevette reconnaissant le collagène de crevette ou le fragment polypeptidique de celui-ci.

6. Utilisation selon la revendication 5, dans lequel l'anticorps allergène anti-crevette est contenu dans une solution.

7. Utilisation selon la revendication 5, dans lequel l'anticorps allergène anti-crevette est supporté dans une matrice de support.

8. Utilisation selon l'une quelconque des revendications 5 à 7, dans lequel l'échantillon est choisi parmi un aliment, une boisson ou un de leurs ingrédients.

9. Procédé de mesure de la sensibilité à un allergène de crevette, comprenant les étapes consistant à :

    faire réagir un échantillon biologique issu d'un sujet, ou une de ses préparations, avec un allergène de crevette comprenant un collagène de crevette isolé ou un fragment polypeptidique de celui-ci ; et
    mesurer une quantité d'un anticorps IgE qui se lie à l'allergène de crevette parmi les anticorps IgE contenus dans l'échantillon biologique ou la préparation de celui-ci.

10. Procédé selon la revendication 9, dans lequel le collagène de crevette isolé a un poids moléculaire de 110 à 140 kDa déterminé par analyse par électrophorèse sur gel de SDS-polyacrylamide.

11. Procédé selon la revendication 9 ou la revendication 10, dans lequel le collagène de crevette isolé est dérivé du *Penaeus japonicus* ou du *Pandalus platyceros.*

12. Utilisation d'un agent comprenant un allergène de crevette comprenant un collagène de crevette isolé ou un fragment polypeptidique de celui-ci pour  mesurer la sensibilité d'un sujet à un allergène de crevette *in vitro.*

13. Utilisation selon la revendication 12, dans laquelle le collagène de crevette isolé a un poids moléculaire de 110 à 140 kDa déterminé par analyse par électrophorèse sur gel de SDS-polyacrylamide.

14. Utilisation selon la revendication 12 ou la revendication 13, dans laquelle le collagène de crevette isolé est dérivé du *Penaeus japonicus* ou du *Pandalus platyceros.*

15. Utilisation selon l'une quelconque des revendications 12 à 14, dans laquelle l'agent comprend en outre un anticorps anti-IgE marqué.

[Figure 1]

PENAEUS JAPONICUS COLLAGEN

M

kDa

210

140 — ABOUT 120 kDA

95

70

55

43

36.5

[Figure 2]

[Figure 3]

[Figure 4]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **YONEYAMA K ; ONO A.** Study of food allergy among university students in Japan. *Allergology International,* 2002, vol. 51, 205-208
- **SHIN NAKAMURA ; YOUJI IIKURA.** Latest food allergy. *Nagaishoten,* 2004, 241-244
- **DAUL CB ; SLATTERY M ; REESE G et al.** Identification of the major brown shrimp (Penaeus aztecus) allergen as the muscle protein tropomyosin. *Int Arch Allergy Immunol,* 1994, vol. 105, 49-55
- **YU CJ ; LIN YF ; CHIANG BL ; CHOW LP.** Proteomics and immunological analysis of a novel shrimp allergen, Pen m 2. *J Immunol.,* 2003, vol. 170, 445-453
- **HAMADA Y ; NAGASIMA Y ; SHIOMI K et al.** Reactivity of IgE in fish-allergic patients to fish muscle collagen. *Allergology International,* 2003, vol. 52, 139-147
- **SAKAGUCHI M ; TODA M ; EBIHARA T et al.** IgE antibody to fish gelatin (type I collagen) in patients with fish allergy. *J Allergy Clin Immunol.,* 2000, vol. 106, 579-584
- Fish and invertebrate collagen. **TETSUYA EBIHARA ; SHINKICHI IRIE.** Method for studying cytology matrix (I. Collagen Technique Workshop, 1998, 52-57